# DEMANDE DE BREVET EUROPEEN

(11) **EP 1 153 580 A1**
(43) Date de publication de la demande: **14.11.2001**
(21) Numéro de dépôt: 00109752.6
(22) Date de dépôt: 09.05.2000
(51) Int. Cl.: A61F 2/06

(54) **Procédé de fabrication d'un implant intravasculaire à déflecteur et implant ainsi obtenu**

(71) Demandeur: EndoArt S.A., 1278 La Rippe (CH)
(72) Inventeur: Imbert, Christian, 1278 La Rippe (CH)
(74) Mandataire: Micheli & Cie

(57) **Abrégé**

L'invention concerne un procédé de fabrication d'un implant vasculaire auto-extensible radialement à déflecteur central (1) ainsi que l'implant en résultant. Le procédé selon l'invention prévoit le découpage d'un cylindre creux de matière de manière à dégager au moins deux bras (3), chacun des bras étant solidaire à l'une de ses extrémités d'une bague de maintien (2). L'extrémité opposée des bras (3) est reliée aux bras adjacents par l'intermédiaire d'un tronçon de matière en zig-zag (7). L'élément de stabilisation ainsi obtenu est ouvert à l'aide d'un outil de formage puis sa forme est fixée par traitement thermique. La bague (2) de l'élément de maintien (2,3) est ensuite fixée sur le déflecteur central (1) pour former l'implant.

## Description

La présente invention concerne un procédé de fabrication d'implants intravasculaires et plus particulièrement des implants intravasculaires auto extensibles radialement et comprenant un corps central permettant une action hémodynamique sur la paroi vasculaire. Un autre objet de l'invention concerne l'implant obtenu par la mise en oeuvre du procédé.

Un implant à déflecteur central est décrit en détail dans la demande de brevet internationale publiée sous le numéro WO 98/58599. Brièvement résumé, ces implants sont destinés au traitement des rétrécissements ou sténoses des vaisseaux sanguins par angioplastie transluminale. Ils comprennent un corps central, maintenu au centre de l'artère par des moyens de maintien. Le déflecteur central dévie le flux sanguin en direction des parois du vaisseau, ce qui permet d'augmenter localement la contrainte de cisaillement sur la paroi de l'artère ou du vaisseau. Grâce à cette action hémodynamique, on a observé que les sténoses se reforment moins fréquemment que lors de l'utilisation de stents traditionnels qui n'ont qu'une action mécanique de support ou de maintien de la paroi artérielle.

Ces implants à déflecteur comportent, comme moyen de maintien et de stabilisation du déflecteur au centre du vaisseau, des spires souples soudées au déflecteur central. Ces spires souples sont extensibles radialement depuis un premier diamètre sensiblement égal au diamètre du déflecteur à un second diamètre supérieur au diamètre de l'artère. Lorsque l'implant est mis en place dans le vaisseau à l'aide d'un cathéter, les spires se déploient radialement et prennent appui contre la paroi interne de l'artère pour maintenir l'implant à l'endroit de la lésion dans le conduit vasculaire. Ces spires souples ont pour fonction d'une part de maintenir le déflecteur au centre de l'artère, afin d'optimiser la déviation du flux sanguin, et d'autre part d'exercer une action mécanique de support sur la paroi artérielle.

On distingue deux sortes d'implants à déflecteur. Une première catégorie d'implants peut être utilisée à l'intérieur d'un stent traditionnel. Dans ce cas, les spires souples ne servent qu'à maintenir et à stabiliser le déflecteur au centre de l'artère pour éviter que le déflecteur entre en contact avec la paroi du vaisseau, l'action de support mécanique sur la paroi vasculaire étant réalisée par le stent préalablement implanté dans le vaisseau sanguin.

La seconde catégorie d'implants, dont les spires sont plus rigides, offre les deux fonctions précédemment décrites à savoir une action hémodynamique grâce au déflecteur et une action de maintien de la paroi vasculaire grâce aux spires souples.

Les implants à déflecteur décrits dans la demande de brevet précitée sont fabriqués de la manière suivante. La partie centrale ou déflecteur est réalisée par enroulement d'un fil rond de manière à former un ressort roulé spire contre spire. L'utilisation d'un ressort pour réaliser le déflecteur donne une souplesse axiale à l'implant et favorise ainsi son acheminement et son implantation dans certains réseaux artériels qui présentent de nombreuses sinuosités. L'organe de maintien du déflecteur a d'abord été réalisé par des spires souples en fil rond. Ces spires sont contraintes dans le cathéter utilisé pour l'implantation, de manière à se déployer radialement lors de la pose de l'implant, favorisant ainsi l'accrochage à la paroi vasculaire et la stabilité du déflecteur au centre de l'artère.

Pour minimiser les micro-turbulences à la surface du déflecteur, ce dernier a été réalisé à partir de fils plats. Les spires souples ont également été réalisées en fils plats pour améliorer la stabilité des bras sur le déflecteur lors du soudage des bras sur le déflecteur central. Des expérimentations animales ont permis de démontrer que les fils servant à la réalisation des bras stabilisateurs, bien que de faibles dimensions, (largeur de 0.18 mm et d'une épaisseur de 0.07 mm) étaient trop forts et que de ce fait l'implant était difficile à manipuler à l'intérieur du cathéter à cause des frottements entre le cathéter et l'implant.

Une fois en place, on a également constaté que les bras réalisés en fils plats, dans les dimensions mentionnées ci-dessus, pouvaient dans certains cas endommager, voire perforer, la paroi vasculaire.

Un autre inconvénient de ce mode de fabrication résulte du fait qu'il n'est pas aisé de souder plusieurs bras indépendants sur la surface du déflecteur central. Il en découle que le centrage du déflecteur n'est pas garanti ou est en tout état de cause loin d'être optimal.

Le procédé objet de l'invention a pour but de remédier aux inconvénients mentionnés ci-dessus et permet de simplifier la fabrication de tels implants.

Les implants obtenus grâce à ce procédé sont plus faciles à mettre en place et garantissent un maintien adéquat du déflecteur au centre du vaisseau. Le risque de lésion ou de perforation de la paroi vasculaire est également considérablement diminué par rapport aux implants fabriqués selon les méthodes exposées précédemment.

L'invention va maintenant être décrite en référence aux dessins annexés qui représentent schématiquement et à titre d'exemple non limitatif deux formes d'exécution d'implants intravasculaires réalisés grâce au procédé selon l'invention.

La figure 1 est une vue en perspective d'un implant à déflecteur central dont l'élément stabilisateur a été réalisé selon le procédé objet de l'invention.

La figure 2 est une vue en perspective d'une deuxième forme d'exécution d'un implant à déflecteur dont l'élément stabilisateur a été réalisé selon le même procédé de fabrication.

La figure 3 est une vue de dessus illustrant les découpes pratiquées dans un cylindre déplié de matière pour réaliser l'élément stabilisateur de l'implant illustré à la figure 2.

La figure 4 est une vue en bout de l'outil de formage utilisé dans la réalisation des implants illustrés aux figures 1 et 2.

La figure 5 est une vue de côté de l'outil de formage illustré à la figure 4.

La figure 6 est une vue en perspective illustrant le formage de l'élément stabilisateur de la figure 2 à l'aide de l'outil illustré à la figure 4.

La figure 7 est une vue en perspective illustrant le formage de l'élément stabilisateur de la figure 1 à l'aide de l'outil illustré à la figure 4.

La figure 8 est une vue en perspective d'une forme d'exécution du déflecteur central de l'implant.

La figure 9 est une vue en coupe d'une variante de réalisation de l'extrémité du déflecteur central.

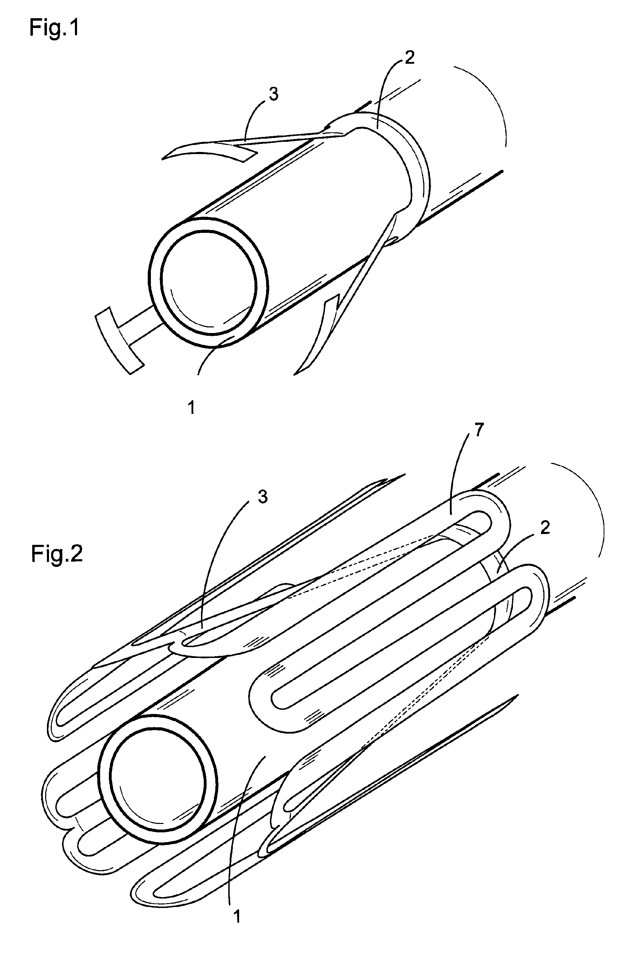

Le procédé objet de l'invention va maintenant être décrit en référence à la figure 1 qui représente un implant vasculaire comportant un déflecteur central 1 sur lequel est fixé un élément stabilisateur comprenant une bague 2 solidaire des bras de stabilisation 3 du déflecteur 1.

Pour obtenir l'élément stabilisateur constitué de la bague 2 et des bras 3, on procède de la manière suivante. La pièce de départ est constituée d'un cylindre de matière creux, dont le diamètre est légèrement supérieur à celui du déflecteur central 1. Ce cylindre de matière est ensuite mis en rotation par exemple sur la broche d'une machine outil, puis découpé à l'aide d'un faisceau laser piloté par ordinateur. La pièce obtenue après découpage comprend une bague 2 solidaire de trois tronçons rectilignes formant les bras de stabilisation 3.

L'extrémité de chacun des bras 3 présente ainsi une forme arrondie s'étendant sur une portion de la circonférence du cylindre original. Cette portion est comprise entre un vingtième et un tiers de la circonférence de la bague 2 de préférence un sixième.

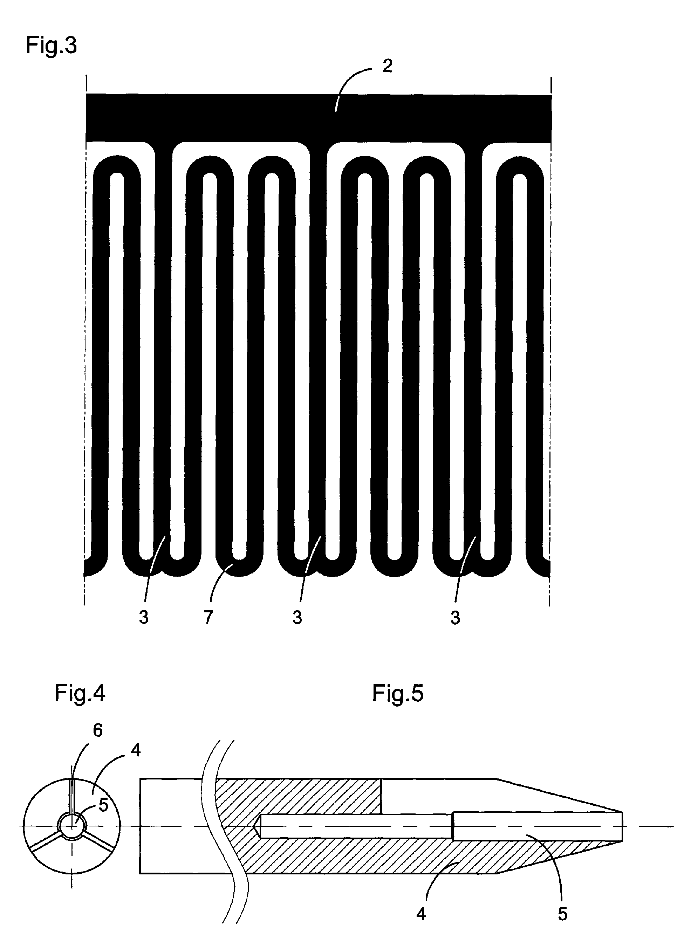

Le cylindre ainsi découpé est ensuite déformé à l'aide d'un outil de formage 4 tel que celui représenté à la figure 5. Cet outil de formage est constitué d'un manchon tronconique qui présente un évidement annulaire longitudinal 5 s'étendant sur une portion de la longueur de l'outil depuis sa partie conique. Le diamètre de l'évidement central 5 est sensiblement supérieur à celui du tube de la bague 2. La partie conique de l'outil 4 comporte trois fentes radiales 6 réparties à 120 degrés et dont la largeur est légèrement supérieure à la largeur des bras 3. Pour faciliter l'usinage de l'outil 4, les fentes radiales peuvent traverser de part en part l'extrémité conique de l'outil 4. Dans ce cas, le nombre de fentes radiales correspond à un multiple du nombre de bras de l'élément stabilisateur.

Le cône de formage est introduit à force par l'extrémité libre du tube découpé jusqu'à ce que la bague 2 pénètre complètement dans l'évidement annulaire 5 de l'outil 4, les bras 3 étant positionnés en regard des fentes radiales.

L'élément stabilisateur ainsi ouvert, à son diamètre maximum, sur le cône de formage, subit ensuite un traitement thermique dont les paramètres dépendent des matériaux utilisés.

Pour réaliser cet élément, on utilisera de préférence des matériaux à durcissement structural fortement allié au cobalt dont la structure moléculaire change grâce audit traitement thermique et donne à la matière une caractéristique ressort incomparable. A titre d'exemple non limitatif, des matériaux tels que le Phynox sont parfaitement adaptés. Dans le cas du Phynox, le traitement thermique s'effectue de préférence à une température comprise entre 480 et 550 degrés Celsius et pendant une durée comprise entre 3 et 4 heures.

D'autres matériaux à mémoire comme le Nitinol peuvent également être utilisés pour réaliser de tels implants. Dans ce cas, les bras sont déformés par pliage à froid ou par formage sur un support prévu à cet effet, puis le traitement thermique s'effectue durant 3 à 4 minutes à une température d'environ 600 degrés Celsius. Grâce à ce procédé de fabrication, on obtient un élément auto-extensible à partir d'un tube de matière découpé par laser.

Ce procédé par découpage laser d'un tube en matériau à durcissement structural suivi d'un traitement thermique adapté permet de réaliser plus simplement et à moindre prix des stents ou des parties de stents qui présentent la caractéristique d'être auto-extensibles.

Une fois la forme de l'élément stabilisateur fixée grâce au traitement thermique, la bague 2 peut être facilement enfilée et solidarisée sur la surface externe du déflecteur central 1 par exemple au moyen d'une soudure laser.

Grâce à ce procédé de fabrication, on obtient un implant dont le déflecteur est parfaitement centré par rapport à l'ensemble stabilisateur. D'autre part, l'extrémité des bras 3 ayant une forme arrondie et de grande surface, puisque constituée d'une portion de cylindre, les surfaces de contact avec la paroi vasculaire sont grandement augmentées par rapport aux stabilisateurs obtenus à partir de fils plats ou ronds, réduisant de ce fait les risques de lésion ou de perforation de la paroi vasculaire. Si l'on désire obtenir la même surface de contact avec un fil plat, ce dernier occupe trop de place et ne peut être introduit dans le cathéter destiné à implanter le dispositif. En revanche l'extrémité des bras 3 de stabilisation obtenus par le procédé décrit a une forme arrondie qui facilite l'introduction de l'implant dans un cathéter standard.

On notera également que grâce à ce procédé de fabrication, on peut travailler avec des bras plus fins que 0.07 millimètres, ce qui représente la limite inférieure pour les fils plats qui en deçà de cette limite ne peuvent plus être correctement soudés sur le déflecteur. Enfin les bras de stabilisation 3 étant solidaires de la bague 2 soudée sur le déflecteur central 1, on ne court plus le risque que les bras ne se désolidarisent du déflecteur central comme cela est le cas dans les réalisations qui prévoient de souder les bras directement sur le déflecteur. L'effort axial exercé sur la bague 2 est négligeable par rapport aux efforts radiaux exercés sur les bras 3.

On notera encore que ce procédé de fabrication permet d'obtenir des implants auto-extensibles, c'est à dire des implants que l'on peut contraindre dans un cathéter et qui se déploient radialement lorsqu'ils sont libérés du cathéter. Les implants auto extensibles connus sont tous fabriqués à partir de structure à fils et non pas à partir d'un tube de matière découpé et déformé.

Les stents connus réalisés par découpe dans un tube de matière ne présentent pas la caractéristique de se déployer radialement automatiquement lors de leur libération, et doivent être déformés mécaniquement, par exemple par gonflement d'un ballonnet, lors de leur mise en place.

L'implant précédemment décrit comporte trois bras 3, ce qui résulte d'un compromis entre une stabilisation adéquate du déflecteur central et le fait que l'on désire minimiser les points de contact des bras sur la paroi vasculaire pour diminuer les risques de blessure. Il est évident que l'on peut prévoir un implant comportant plus de bras, par exemple de quatre à six, sans sortir du cadre de l'invention. Il suffit pour ce faire de pratiquer des découpes supplémentaires dans le cylindre creux et d'adapter l'outil de formage, en particulier en prévoyant un nombre de fentes radiales équivalent au nombre ou à un multiple du nombre de bras souhaités. Il est également envisageable de réduire à deux le nombre de bras de stabilisation, toutefois le maintien du déflecteur 1 au centre du vaisseau ou de l'artère n'est pas optimal dans ce cas de figure.

La figure 2 illustre un implant à déflecteur central dont les bras de stabilisation 3 permettent de maintenir le déflecteur central au centre du vaisseau et d'exercer une action mécanique de maintien sur la paroi vasculaire. L'élément de stabilisation et de maintien est également obtenu à partir d'un cylindre de matière creux. La figure 3 est une vue du cylindre de matière découpé. Pour faciliter la visualisation de la découpe, le cylindre de matière creux est représenté comme si on l'avait découpé longitudinalement puis déroulé et mis à plat. On remarque sur la partie supérieure une zone non découpée qui correspond à la bague de fixation 2 destinée à être soudée sur le déflecteur central. La découpe au laser est pratiquée dans ce cylindre creux de manière à former trois bras 3. Chacun des bras 3 est relié aux bras adjacents par l'intermédiaire d'un segment de matière 7 présentant une configuration en zigzag. Sur l'exemple représenté, chacun des trois bras 3 est relié aux deux autres bras adjacents par un segment en zigzag 7 présentant deux aller-retour entre la bague 2 et l'extrémité du cylindre, soit quatre segments rectilignes connectés par un segment courbe. Dans une variante, on peut prévoir d'autres configurations, par exemple en augmentant ou en diminuant le nombre d'aller-retour du tronçon en zigzag 7 ou en augmentant ou en réduisant le nombre de bras 3.

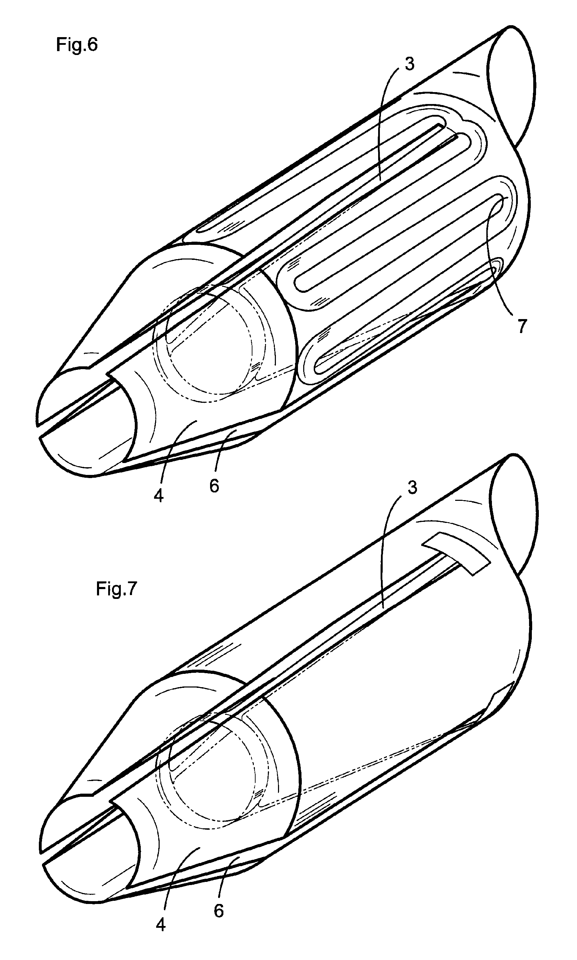

Une fois cette découpe réalisée dans le cylindre de matière, et comme précédemment décrit, l'élément de maintien et de stabilisation est déformé à l'aide de l'outil de formage 4. La figure 6 illustre l'élément de stabilisation ouvert à son diamètre maximum. On voit les bras 3 logés dans les fentes radiales 6 du cône de formage 4, la bague 2 étant à l'intérieur de l'évidement central 5 de l'outil 4. Dans cette position, l'élément stabilisateur est prêt pour le traitement thermique qui va fixer sa forme définitive. Après le traitement thermique, l'élément de maintien du déflecteur central présente la configuration illustrée à la figure 2. On procède ensuite à l'assemblage de l'élément stabilisateur en fixant la bague 2 sur le corps du déflecteur central 1. Grâce à cette configuration, l'élément stabilisateur remplit deux fonctions différentes. Il permet de maintenir le déflecteur au centre du vaisseau et exerce à l'aide du tronçon en zigzag 7 une action de maintien mécanique sur la paroi vasculaire comme un stent traditionnel.

La figure 7 illustre de façon analogue l'implant illustré à la figure 1 déformé par l'outil de formage 4 pour permettre son traitement thermique.

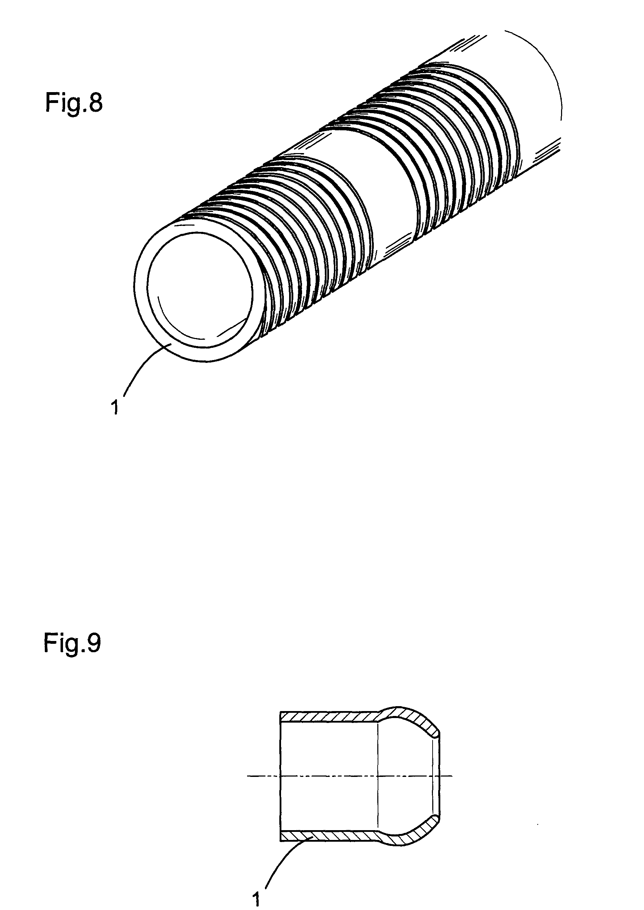

La figure 8 illustre une forme de réalisation du déflecteur central également obtenu à partir d'un tube de matière dans lequel on pratique une découpe hélicoïdale. On obtient ainsi un ressort dont l'aspect en surface est moins rugueux que celui d'un ressort réalisé à l'aide d'un fil plat. La découpe hélicoïdale est interrompue sur une partie du déflecteur, à l'endroit où la bague 2 de l'élément stabilisateur est fixée. Dans une variante, la découpe hélicoïdale peut s'étendre sur toute la longueur du déflecteur.

A la figure 9, on a représenté en coupe l'extrémité d'un déflecteur qui à été déformé par un outil de roulage pour présenter une partie conique à l'une de ses extrémités. Lors de l'implantation, ce cône émergeant de la partie distale du cathéter favorise l'introduction de l'implant dans les réseaux artériels de faible diamètre et présentant de nombreuses sinuosités.

## Revendications

1. Procédé de fabrication d'un implant vasculaire auto extensible radialement, **caractérisé par le fait qu'**il comporte les étapes suivantes:
- découpage d'un cylindre creux en un matériau à durcissement structural,
- déformation de l'élément ainsi obtenu de manière à ouvrir au moins une portion du cylindre découpé à un diamètre supérieur à celui du conduit vasculaire dans lequel il est destiné à être implanté,
- traitement thermique de l'élément déformé à son diamètre maximum pour en fixer la forme.

2. Procédé de fabrication d'un implant selon la revendication 1, **caractérisé par le fait que** le découpage du cylindre est effectué de manière à dégager au moins deux bras longitudinaux (3), chacun des bras (3) étant solidaire à l'une de ses extrémités d'une bague (2) de maintien et libre à son extrémité opposée et **par le fait que** l'élément de stabilisation ainsi obtenu est déformé de manière à ce que l'extrémité libre des bras (3) soit située sur un diamètre supérieur à celui du conduit vasculaire dans lequel il doit être implanté.

3. Procédé de fabrication d'un implant vasculaire selon la revendication 2, **caractérisé par le fait que** l'on fixe l'élément de stabilisation au moyen de la bague (2) sur la surface externe d'un déflecteur central (1).

4. Procédé de fabrication selon l'une des revendications précédentes, **caractérisé par le fait que** l'on déforme l'élément de stabilisation découpé (2,3) en introduisant par son extrémité libre un outil de formage tronconique (4) présentant un évidement central longitudinal (5) d'un diamètre correspondant à celui du tube de matière, ainsi qu'un nombre de fentes radiales (6), dans sa partie conique, correspondant au nombre de bras de stabilisation (3).

5. Implant vasculaire comprenant un déflecteur de flux (1) permettant de dévier les lignes de courant radialement en direction des parois artérielles, et des moyens de maintien prenant appui en position de service contre la surface interne du vaisseau, **caractérisé par le fait que** les moyens de maintien sont constitués d'au moins deux bras (3) solidaires à l'une de leur extrémité d'une bague (2) destinée à être fixée sur le déflecteur (1) et **par le fait que** l'extrémité libre de chacun des bras (3) est située sur une circonférence d'un diamètre supérieur à celui de la bague (2).

6. Implant vasculaire selon la revendication 4, **caractérisé par le fait qu'**il comporte trois bras (3) répartis uniformément sur la circonférence de la bague (2) l'extrémité libre des bras (3) présentant une forme arrondie s'étendant sur une portion de la circonférence du cylindre découpé comprise entre un vingtième et un tiers de la circonférence de la bague (2), de préférence un sixième.

7. Implant vasculaire selon la revendication 4, **caractérisé par le fait que** chacun des bras (3) est relié, par son extrémité libre, aux bras (3) adjacents par un tronçon de matière (7) présentant une configuration en zigzag.

8. Implant vasculaire selon la revendication 5, **caractérisé par le fait que** le tronçon de matière (7) en zigzag reliant l'extrémité libre de deux bras (3) adjacents présente au moins un aller-retour entre l'extrémité libre du bras (3) et la bague (2).

9. Implant vasculaire selon l'une des revendications 3 à 6, **caractérisé par le fait que** le déflecteur central (1) est constitué d'un tube de matière présentant sur une partie au moins de sa longueur une découpe hélicoïdale.

10. Outil de formage pour la mise en oeuvre du procédé de fabrication selon la revendication 1, **caractérisé par le fait qu'**il est constitué d'un manchon tronconique (4) présentant un évidement longitudinal central (5) apte à recevoir un élément stabilisateur obtenu à partir d'un cylindre creux de matière découpé et **par le fait qu'**il présente dans sa partie conique un nombre de fentes radiales (6) correspondant à un multiple du nombre de bras (3) de l'élément stabilisateur.
